# EUROPEAN PATENT APPLICATION

(11) **EP 4 628 120 A2**
(43) Date of publication of application: **08.10.2025**
(21) Application number: 25162591.9
(22) Date of filing: 10.03.2025
(51) Int. Cl.: A61M 1/36, A61M 1/16, A61M 1/32

(54) **PRIMING SYSTEM FOR USE WITH A MEDICAL FLUID SYSTEM**

(30) Priority: 02.04.2024 US 202418624539
(71) Applicant: CardiacAssist, Inc., Pittsburgh, PA 15238 (US)
(72) Inventor: DACEY, Chris, Barrington, RI 02806 (US)
(74) Representative: Buzzi, Notaro & Antonielli d'Oulx S.p.A.

(57) **Abstract**

A system for priming a fluid system includes a fluid connector configured to interface with a fluid source and a fluid reservoir including a first connector configured to couple with an inflow line of the fluid system, a second connector configured to couple with an outflow line of the fluid system, and a gas release valve. A system for priming a fluid system includes first and second fluid connectors configured to interface with a fluid source, a first connector configured to couple with an inflow line of the fluid system, a second connector configured to couple with an outflow line of the fluid system, and a gas release valve. A method of priming the fluid system includes coupling the first connector with the inflow line, coupling the second connector with the outflow line, coupling the first fluid connector with the fluid source, and elevating the fluid source.

## Description

### TECHNICAL FIELD

The present disclosure relates generally to extracorporeal circulation. More particularly, the present disclosure relates to systems and methods for priming medical fluid systems such as extracorporeal life support systems and/or subsystems thereof.

### BACKGROUND

In some Extracorporeal Life Support (ECLS) systems, the oxygenator and pump are separate components. In some ECLS systems, there are subsystems that must be primed to remove any entrained air or gases prior to use and/or prior to connecting the subsystem to the overall ECLS system. Some existing priming tools utilize a secondary reservoir that requires a volume of fluid to be transferred therein, thereby adding steps, devices, components, and/or complexity to the preparation of the subsystem before use.

There is an ongoing need for alternative ECLS systems, devices, components, and/or methods of use and/or manufacture of said systems, devices, and/or components.

### SUMMARY

In one example, a system for priming a medical fluid system may comprise a fluid connector configured to interface with a sterile fluid source, and a fluid reservoir downstream of and in fluid communication with the fluid connector. The fluid reservoir may comprise a first connector configured to fluidly couple with an inflow line of the medical fluid system, a second connector configured to fluidly couple with an outflow line of the medical fluid system, and a gas release valve configured to permit gas to pass therethrough and prevent fluid from passing therethrough.

In addition, or alternatively, to any example disclosed herein, the fluid reservoir further comprises a one-way baffle disposed between the second connector and the first connector.

In addition, or alternatively, to any example disclosed herein, the one-way baffle permits fluid to flow therethrough from a second connector side of the one-way baffle toward a first connector side of the one-way baffle.

**In** addition, or alternatively, to any example disclosed herein, the gas release valve is upstream of the one-way baffle.

**In** addition, or alternatively, to any example disclosed herein, the one-way baffle permits fluid to flow therethrough only from the second connector side of the one-way baffle toward the first connector side of the one-way baffle.

**In** addition, or alternatively, to any example disclosed herein, the fluid reservoir is configured to recirculate fluid from the sterile fluid source through the medical fluid system fluidly coupled thereto.

**In** addition, or alternatively, to any example disclosed herein, the gas release valve is configured to vent gas to atmosphere and direct fluid into the fluid reservoir.

In addition, or alternatively, to any example disclosed herein, the system may further comprise a fluid supply line extending from the first fluid connector to the fluid reservoir.

In addition, or alternatively, to any example disclosed herein, the medical fluid system may comprise a fluid pump and an oxygenator.

In addition, or alternatively, to any example disclosed herein, the sterile fluid source is a saline bag.

In addition, or alternatively, to any example disclosed herein, and in a second example, a method of priming a medical fluid system may comprise: coupling a first connector of a fluid reservoir with an inflow line of the medical fluid system; coupling a second connector of the fluid reservoir with an outflow line of the medical fluid system; coupling a first fluid connector with a sterile fluid source, wherein the first fluid connector is in fluid communication with the fluid reservoir; and elevating the sterile fluid source above the medical fluid system, thereby causing fluid from the sterile fluid source to flow through the fluid reservoir into the inflow line of the medical fluid system, and through the medical fluid system thereby pushing gas disposed therein downstream through the outflow line to a gas release valve. The gas release valve may be configured to vent gas to atmosphere and direct fluid into the fluid reservoir.

In addition, or alternatively, to any example disclosed herein, the method may further comprise recirculating the fluid in the fluid reservoir through the medical fluid system.

In addition, or alternatively, to any example disclosed herein, and in a third example, a system for priming a medical fluid system may comprise a first adapter assembly comprising a first fluid connector configured to interface with a sterile fluid source and a first connector configured to fluidly couple with an inflow line of the medical fluid system, and a second adapter assembly comprising a second fluid connector configured to interface with the sterile fluid source, a second connector configured to fluidly couple with an outflow line of the medical fluid system, and a gas release valve configured to permit gas to pass therethrough and prevent fluid from passing therethrough.

In addition, or alternatively, to any example disclosed herein, the gas release valve is disposed upstream of the second fluid connector.

In addition, or alternatively, to any example disclosed herein, the gas release valve is disposed between the second fluid connector and the second connector.

In addition, or alternatively, to any example disclosed herein, the gas release valve is configured to vent gas to atmosphere and direct fluid to the second fluid connector.

In addition, or alternatively, to any example disclosed herein, the medical fluid system may comprise a fluid pump and an oxygenator.

In addition, or alternatively, to any example disclosed herein, the sterile fluid source is a saline bag.

In addition, or alternatively, to any example disclosed herein, and in a fourth example, a method of priming a medical fluid system may comprise: coupling a first connector of a first adapter assembly with an inflow line of the medical fluid system; coupling a second connector of a second adapter assembly with an outflow line of the medical fluid system; coupling a first fluid connector of the first adapter assembly with a sterile fluid source; coupling a second fluid connector of the second adapter assembly with the sterile fluid source; and elevating the sterile fluid source above the medical fluid system, thereby causing fluid from the sterile fluid source to flow into the inflow line of the medical fluid system and through the medical fluid system thereby pushing gas disposed therein downstream through the outflow line to a gas release valve. The gas release valve may be configured to vent gas to atmosphere and direct fluid into the sterile fluid source.

In addition, or alternatively, to any example disclosed herein, the method may further comprise recirculating the fluid directed into the sterile fluid source by the gas release valve through the medical fluid system.

The above summary of some embodiments, aspects, and/or examples is not intended to describe each disclosed embodiment or every implementation of the present disclosure. The figures and detailed description which follow more particularly exemplify these embodiments.

### BRIEF DESCRIPTION OF THE DRAWINGS

The disclosure may be more completely understood in consideration of the following detailed description in connection with the accompanying drawings, in which:
FIG. 1 illustrates selected aspects of a medical fluid system and a system for priming the medical fluid system;
FIG. 2 illustrates selected aspects of the system of FIG. 1 and methods of use;
FIG. 3 illustrates selected aspects of a medical fluid system and a system for priming the medical fluid system; and
FIG. 4 illustrates selected aspects of the system of FIG. 3 and methods of use.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### DETAILED DESCRIPTION

The following description should be read with reference to the drawings, which are not necessarily to scale, wherein like reference numerals indicate like elements throughout the several views. The detailed description and drawings are intended to illustrate example embodiments of the disclosure but not limit the disclosure. Those skilled in the art will recognize that the various elements described and/or shown may be arranged in various combinations and configurations without departing from the scope of the disclosure.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5). Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. It will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Still other relative terms, such as "axial", "circumferential", "longitudinal", "lateral", "radial", etc. and/or variants thereof generally refer to direction and/or orientation relative to a central longitudinal axis of the disclosed structure or device.

The term "extent" may be understood to mean the greatest measurement of a stated or identified dimension, unless the extent or dimension in question is preceded by or identified as a "minimum", which may be understood to mean the smallest measurement of the stated or identified dimension. For example, "outer extent" may be understood to mean an outer dimension, "radial extent" may be understood to mean a radial dimension, "longitudinal extent" may be understood to mean a longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered the greatest possible dimension measured according to the intended usage, while a "minimum extent" may be considered the smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete structures or elements together.

For the purpose of this disclosure, the term "fluid" shall be understood to include liquid(s) and/or liquid states, including but not limited to suspensions and/or liquid compounds, and shall exclude gas(es) and/or gaseous states. The term "gas" may include elemental gases, gas mixtures such as air and/or gaseous compounds, and/or vapors.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to use the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

Additionally, it should be noted that in any given figure, some features may not be shown, or may be shown schematically, for clarity and/or simplicity. Additional details regarding some components and/or method steps may be illustrated in other figures in greater detail. The devices and/or methods disclosed herein may provide a number of desirable features and benefits as described in more detail below.

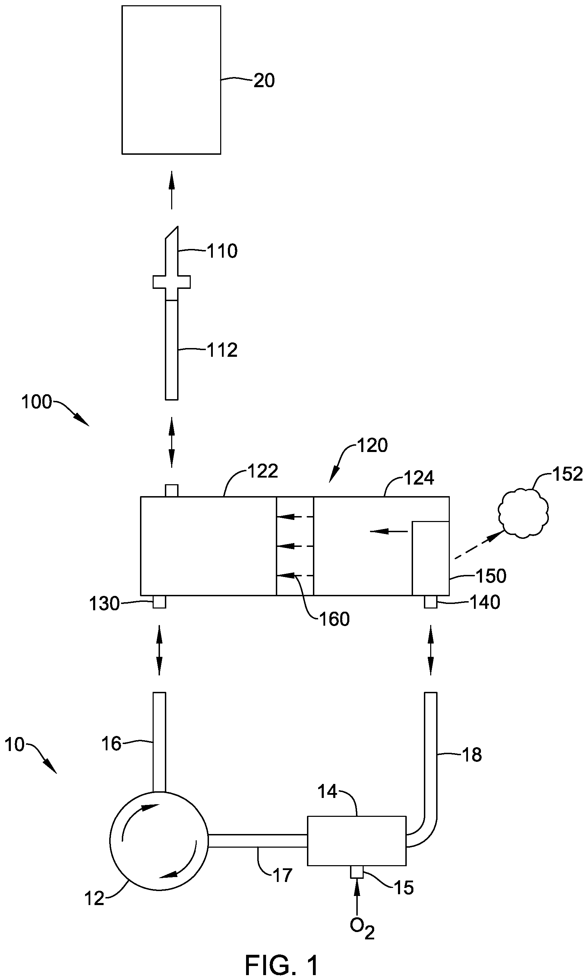

FIG. 1 illustrates, in part, selected aspects of a system 100 for priming a medical fluid system. FIG. 1 also illustrates one example configuration of a medical fluid system 10. In some embodiments, the medical fluid system 10 may be an extracorporeal life support (ECLS) system, or a subsystem or component of an ECLS system. In some embodiments, the medical fluid system 10 may comprise a fluid pump 12, and an oxygenator 14. In some embodiments, the oxygenator 14 may be separate from the fluid pump 12. In some alternative embodiments, the fluid pump 12 may be integrated into the oxygenator 14 or the oxygenator 14 may be integrated into the fluid pump 12. In some embodiments, the medical fluid system 10 may comprise tubing forming an inflow line 16, an outflow line 18, and a transfer line 17. In some embodiments, the inflow line 16 may be fluidly coupled to and extend upstream from the fluid pump 12. In some embodiments, during use, the inflow line 16 may be configured to fluidly couple with a venous blood circuit (e.g., a patient's vein). In some embodiments, the outflow line 18 may be fluidly coupled to and extend downstream from the oxygenator 14. In some embodiments, during use, the outflow line 18 may be configured to fluidly coupled with an arterial blood circuit (e.g., a patient's artery). In some embodiments, the transfer line 17 may fluidly couple the fluid pump 12 to the oxygenator 14 for transporting fluid from the fluid pump 12 to the oxygenator 14. In at least some embodiments, the inflow line 16, the outflow line 18, and/or the transfer line 17 may be formed from 0.375-inch (9.525 millimeter) polymeric tubing. Other tubing sizes are also possible.

In some instances, the medical fluid system 10 may comprise and/or may be coupled to and/or integrated into a wearable support garment (e.g., a strap or belt, a vest such as the VoyagerVest^{®} from CardiacAssist, Inc., etc.), thereby rendering the medical fluid system 10 more portable than systems utilizing a trolley. In some embodiments, the fluid pump 12 may be coupled to and/or received by a mount secured to the oxygenator 14 and/or the wearable support garment. In some embodiments, the oxygenator 14 may be secured to the wearable support garment. In some embodiments, the wearable support garment may include clips and/or straps to secure the tubing (e.g., the inflow line 16, the outflow line 18, etc.) to the wearable support garment.

In addition, or alternatively, the medical fluid system 10 may be and/or may include other types, components, and/or subsystems of medical fluid systems including but not limited to fluid management systems, irrigation systems, etc. In some embodiments, the medical fluid system 10 may include centrifugal pumping apparatuses and/or closed (e.g., non-auto-venting) systems that require atmospheric displacement. Other configurations are also contemplated.

In some embodiments, the oxygenator 14 may comprise a gas inlet 15. The gas inlet 15 may be configured to connect to a gas supply source via polymeric tubing (not shown) to transport gas (e.g., oxygen, air, etc.) to the oxygenator 14. The oxygenator 14 may comprise a gas outlet port (not shown) configured to transport gas (e.g., oxygen, air, etc.) away from the oxygenator 14. During use, gas may be transferred between the oxygenator 14 and blood flowing through the oxygenator 14 (e.g., oxygen into the blood, carbon dioxide out of the blood, etc.).

In some embodiments, the medical fluid system 10 and/or the system 100 for priming a medical fluid system may include one or more integrated blood sensors (not shown). For example, the one or more integrated blood sensors may be configurated to monitor and/or measure pressure, oxygen saturation, temperature, the presence of air bubbles, etc. Other sensors and/or configurations are also contemplated. In some embodiments, the one or more integrated blood sensors may improve control and/or reduce complexity of the medical fluid system 10 and/or the system 100 for priming a medical fluid system.

The medical fluid system 10 may define a total volume of extracorporeal blood and/or fluid. In some embodiments, the total volume of the medical fluid system 10 may depend on patient size. For example, some medical fluid systems may be designed for use with adult patients or for use with pediatric patients. Other configurations are also contemplated. Larger patients generally require a larger total volume of fluid and thus a larger volume of priming fluid. By reducing the number of components involved in priming the medical fluid system 10, priming volume can also be reduced. In some embodiments, recirculating fluid during priming rather than introducing new fluid may also reduce the volume of fluid required for priming.

In some embodiments, the system 100 for priming a medical fluid system may comprise a fluid connector 110 configured to interface with a sterile fluid source 20. In at least some embodiments, the sterile fluid source 20 may be and/or may include a saline IV bag. Other configurations are also contemplated.

In some embodiments, the system 100 for priming a medical fluid system may comprise a fluid reservoir 120 downstream of and in fluid communication with the fluid connector 110. In some embodiments, the system 100 for priming a medical fluid system may comprise a fluid supply line 112 extending from the fluid connector 110 to the fluid reservoir 120. In some embodiments, the fluid supply line 112 may be formed from 0.375-inch (9.525 millimeter) polymeric tubing. Other tubing sizes are also possible. In some embodiments, the fluid connector 110 may be coupled directly to the fluid reservoir 120. In some embodiments, the fluid connector 110 may be monolithically formed with the fluid reservoir 120. Other configurations are also contemplated.

In some embodiments, the fluid reservoir 120 may comprise a first connector 130 configured to fluidly couple with an inflow line of a medical fluid system, such as the inflow line 16 of the medical fluid system 10. In some embodiments, the first connector 130 may be coupled directly to the fluid reservoir 120. In some embodiments, the first connector 130 may be monolithically formed with the fluid reservoir 120. In some embodiments, the first connector 130 may be spaced apart from the fluid reservoir 120 by tubing fluidly coupling the first connector 130 with the fluid reservoir 120. Other configurations are also contemplated.

In some embodiments, the fluid reservoir 120 may comprise a second connector 140 configured to fluidly couple with an outflow line of a medical fluid system, such as the outflow line 18 of the medical fluid system 10. In some embodiments, the second connector 140 may be coupled directly to the fluid reservoir 120. In some embodiments, the second connector 140 may be monolithically formed with the fluid reservoir 120. In some embodiments, the second connector 140 may be spaced apart from the fluid reservoir 120 by tubing fluidly coupling the second connector 140 with the fluid reservoir 120. Other configurations are also contemplated.

In some embodiments, the system 100 for priming a medical fluid system and/or the fluid reservoir 120 may comprise a gas release valve 150 configured to permit gas (e.g., air, oxygen, carbon dioxide, etc.) to pass therethrough and prevent fluid (e.g., liquid) from passing therethrough. In some embodiments, the gas release valve 150 may be configured to vent gas 152 (e.g., air, oxygen, carbon dioxide, etc.) to atmosphere and direct fluid (e.g., liquid) into the fluid reservoir 120. In some alternative embodiments, the gas release valve 150 may be configured to vent gas (e.g., air, oxygen, carbon dioxide, etc.) into a collection container (not shown) and direct fluid (e.g., liquid) into the fluid reservoir 120. In some embodiments, the collection container may be separate from the fluid reservoir 120.

In some embodiments, the gas release valve 150 may be disposed within the fluid reservoir 120. In some embodiments, the gas release valve 150 may be coupled directly to the fluid reservoir 120. In some embodiments, the gas release valve 150 may be integrated into the fluid reservoir 120. In some embodiments, the gas release valve 150 may be disposed within a wall of the fluid reservoir 120.

In some embodiments, the fluid reservoir 120 may comprise a one-way baffle 160 disposed therein. In some embodiments, the fluid reservoir 120 may comprise a first chamber 122 and a second chamber 124. In some embodiments, the one-way baffle 160 may divide the fluid reservoir 120 into the first chamber 122 and the second chamber 124. In some embodiments, the one-way baffle 160 may be disposed between the first chamber 122 and the second chamber 124. In some embodiments, the one-way baffle 160 may at least partially define the first chamber 122 and/or the second chamber 124.

In some embodiments, the first fluid connector and/or the first connector 130 may be in fluid communication with the first chamber 122. In some embodiments, the second connector 140 and/or the gas release valve 150 may be in fluid communication with the second chamber 124. In some embodiments, the one-way baffle 160 may be disposed between the first connector 130 and the second connector 140. In some embodiments, the first chamber 122 may be disposed on a first connector side of the one-way baffle 160 and the second chamber 124 may be disposed on a second connector side of the one-way baffle 160. In some embodiments, the one-way baffle 160 may be disposed between the first connector 130 and the gas release valve 150.

In some embodiments, the one-way baffle 160 may be configured to permit fluid (e.g., liquid) to flow or pass therethrough in a first direction. In some embodiments, the one-way baffle 160 may be configured to permit fluid (e.g., liquid) to flow or pass therethrough only in the first direction. In some embodiments, the one-way baffle 160 may be configured to prevent fluid (e.g., liquid) from flowing or passing therethrough in a second direction opposite the first direction. In some embodiments, the one-way baffle 160 may be configured to prevent gas (e.g., air, oxygen, carbon dioxide, etc.) from flowing or passing therethrough. In some embodiments, the one-way baffle 160 may be configured to prevent gas (e.g., air, oxygen, carbon dioxide, etc.) from flowing or passing therethrough in the first direction and/or the second direction.

In some embodiments, the one-way baffle 160 may be configured to permit fluid (e.g., liquid) to flow or pass therethrough from the second chamber 124 toward and/or to the first chamber 122. In some embodiments, the one-way baffle 160 may be configured to permit fluid (e.g., liquid) to flow or pass therethrough only from the second chamber 124 to the first chamber 122. In some embodiments, the one-way baffle 160 may be configured to prevent fluid (e.g., liquid) from flowing or passing therethrough from the first chamber 122 toward and/or to the second chamber 124. In some embodiments, the one-way baffle 160 may be configured to permit fluid (e.g., liquid) to flow or pass therethrough from the second connector side of the one-way baffle 160 toward and/or to the first connector side of the one-way baffle 160. In some embodiments, the one-way baffle 160 may be configured to permit fluid (e.g., liquid) to flow or pass therethrough only from the second connector side of the one-way baffle 160 toward and/or to the first connector side of the one-way baffle 160.

In some embodiments, the fluid reservoir 120 may be configured to recirculate fluid from the sterile fluid source 20 through a medical fluid system fluidly coupled thereto, such as the medical fluid system 10 fluidly coupled to the fluid reservoir 120 via the first connector 130 and the second connector 140. For example, as fluid from the sterile fluid source 20 passes through the first chamber 122 of the fluid reservoir 120 into the inflow line 16, the fluid may displace and/or drive gas (e.g., air, etc.) disposed within the medical fluid system 10 and/or components thereof, such as the inflow line 16, the fluid pump 12, the oxygenator 14, the outflow line 18, etc., downstream toward and/or to the gas release valve 150. The gas release valve 150 may be configured to vent the gas (e.g., air, etc.) out of the medical fluid system 10 and/or the system 100 for priming a medical fluid system and direct the fluid into the fluid reservoir 120. As fluid accumulates in the fluid reservoir 120, the fluid may be recirculated through the medical fluid system 10 so that additional fluid (and/or additional sterile fluid sources) is not required to prime, and/or properly remove gas (e.g., air, etc.) from, the medical fluid system 10, thereby reducing complexity for the user, as well as reducing cost and waste.

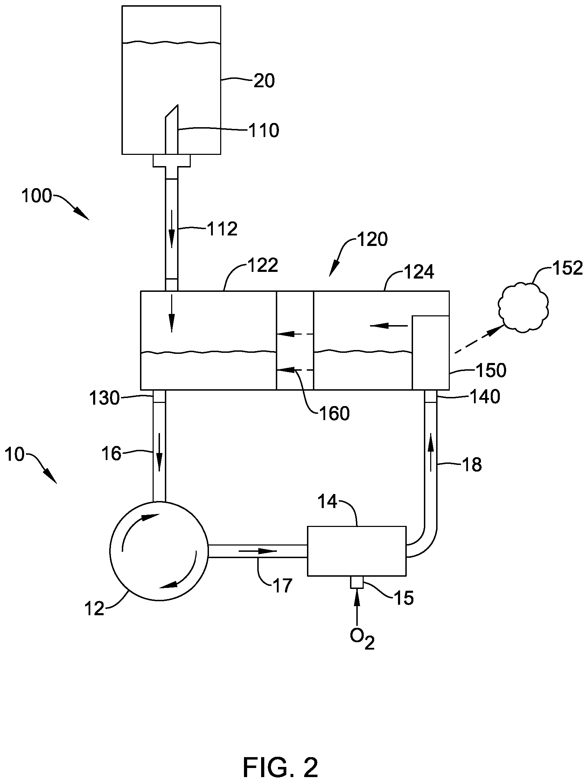

FIG. 2 illustrates selected aspects of the system 100 for priming a medical fluid system in use with the medical fluid system 10 and selected aspects of a method of priming a medical fluid system, such as the medical fluid system 10.

In some embodiments, the method of priming the medical fluid system 10 may comprise coupling the first connector 130 of the fluid reservoir 120 with the inflow line 16 of the medical fluid system 10. In some embodiments, the first connector 130 may comprise a female connector and coupling the first connector 130 of the fluid reservoir 120 with the inflow line 16 of the medical fluid system 10 may comprise inserting the inflow line 16 of the medical fluid system 10 into the first connector 130. In some embodiments, the first connector 130 may comprise a male connector and coupling the first connector 130 of the fluid reservoir 120 with the inflow line 16 of the medical fluid system 10 may comprise advancing and/or positioning the inflow line 16 of the medical fluid system 10 onto the first connector 130. In some embodiments, the inflow line 16 of the medical fluid system 10 may comprise a first mating connector configured to matingly couple with the first connector 130. Other configurations are also contemplated.

In some embodiments, the method of priming the medical fluid system 10 may comprise coupling the second connector 140 of the fluid reservoir 120 with the outflow line 18 of the medical fluid system 10. In some embodiments, the second connector 140 may comprise a female connector and coupling the second connector 140 of the fluid reservoir 120 with the outflow line 18 of the medical fluid system 10 may comprise inserting the outflow line 18 of the medical fluid system 10 into the second connector 140. In some embodiments, the second connector 140 may comprise a male connector and coupling the second connector 140 of the fluid reservoir 120 with the outflow line 18 of the medical fluid system 10 may comprise advancing and/or positioning the outflow line 18 of the medical fluid system 10 onto the second connector 140. In some embodiments, the outflow line 18 of the medical fluid system 10 may comprise a second mating connector configured to matingly couple with the second connector 140. Other configurations are also contemplated.

In some embodiments, the method of priming the medical fluid system 10 may comprise coupling the fluid connector 110 of the system 100 for priming a medical fluid system with the sterile fluid source 20. In some embodiments, coupling the fluid connector 110 with the sterile fluid source 20 may comprise inserting the fluid connector 110 into the sterile fluid source 20. In some embodiments, coupling the fluid connector 110 with the sterile fluid source 20 may comprise coupling the fluid connector 110 with a first mating fluid connector of and/or in fluid communication with the sterile fluid source 20. Other configurations are also contemplated.

In some embodiments, the method of priming the medical fluid system 10 may comprise elevating the sterile fluid source 20 above the medical fluid system 10, thereby causing fluid from the sterile fluid source 20 to flow through the fluid reservoir 120 into the inflow line 16 of the medical fluid system 10, and through the medical fluid system 10 thereby pushing gas disposed therein downstream through the outflow line 18 to the gas release valve 150. In some embodiments, the sterile fluid source 20 may be disposed and/or elevated above the medical fluid system 10 before coupling the fluid connector 110 with the sterile fluid source 20 such that gravity immediately causes fluid to flow from the sterile fluid source 20 toward and/or through the fluid reservoir 120 as soon as the fluid connector 110 is coupled with the sterile fluid source 20 without any further interaction by the user/practitioner. For example, the sterile fluid source 20 may be a saline IV bag disposed and/or hanging on a support hook adjacent the patient when the fluid connector 110 is coupled with the sterile fluid source 20. In some embodiments, the sterile fluid source 20 may be disposed and/or elevated above the medical fluid system 10 after coupling the fluid connector 110 with the sterile fluid source 20 such that the user/practitioner must elevate the sterile fluid source 20 after coupling the fluid connector 110 with the sterile fluid source 20 in order to initiate fluid flow from the sterile fluid source 20 toward and/or through the fluid reservoir 120. Other configurations are also contemplated.

In some embodiments, the gas release valve 150 may be configured to vent gas 152 (e.g., air, oxygen, carbon dioxide, etc.) to atmosphere and direct fluid (e.g., liquid) into the fluid reservoir 120. In some embodiments, the method of priming the medical fluid system 10 may comprise venting gas 152 (e.g., air, oxygen, carbon dioxide, etc.) to atmosphere or a collection container with the gas release valve 150. In some embodiments, the method of priming the medical fluid system 10 may comprise directing fluid (e.g., liquid) into the fluid reservoir 120 with the gas release valve 150. Other configurations are also contemplated.

In some embodiments, the method of priming the medical fluid system 10 may comprise recirculating the fluid in the fluid reservoir 120 through the medical fluid system 10. As discussed herein, as fluid flows from the sterile fluid source 20 through the medical fluid system 10, fluid is directed into the second chamber 124 of the fluid reservoir 120 by the gas release valve 150. Fluid in the second chamber 124 of the fluid reservoir 120 may flow and/or pass through the one-way baffle 160 into the first chamber 122 of the fluid reservoir 120, where the fluid may flow into the inflow line 16 of the medical fluid system 10, thereby recirculating the fluid in the fluid reservoir 120 through the medical fluid system 10.

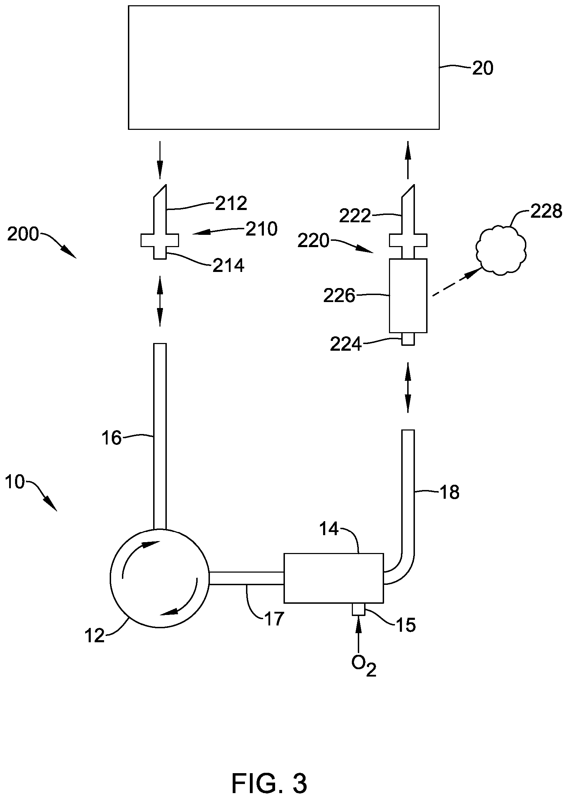

FIG. 3 illustrates, in part, selected aspects of a system 200 for priming a medical fluid system, along with the medical fluid system 10, which is described above and therefore details of the medical fluid system 10 are not repeated. Similar to above, in some embodiments, the medical fluid system 10 and/or the system 200 for priming a medical fluid system may include one or more integrated blood sensors (not shown). For example, the one or more integrated blood sensors may be configurated to monitor and/or measure pressure, oxygen saturation, temperature, the presence of air bubbles, etc. Other sensors and/or configurations are also contemplated. In some embodiments, the one or more integrated blood sensors may improve control and/or reduce complexity of the medical fluid system 10 and/or the system 200 for priming a medical fluid system.

In some embodiments, the system 200 for priming a medical fluid system may comprise a first adapter assembly 210 comprising a first fluid connector 212 configured to interface with a sterile fluid source 20 and a first connector 214 configured to fluidly couple with an inflow line of a medical fluid system, such as the inflow line 16 of the medical fluid system 10. As discussed herein, in at least some embodiments, the sterile fluid source 20 may be and/or may include a saline IV bag. Other configurations are also contemplated.

In some embodiments, the first fluid connector 212 may be coupled directly to the first adapter assembly 210. In some embodiments, the first fluid connector 212 may be monolithically formed with the first adapter assembly 210. In some alternative embodiments, the first fluid connector 212 may be spaced apart from the first adapter assembly 210 by tubing fluidly coupling the first fluid connector 212 with the first adapter assembly 210.

In some embodiments, the first connector 214 may be coupled directly to the first adapter assembly 210. In some embodiments, the first connector 214 may be monolithically formed with the first adapter assembly 210. In some alternative embodiments, the first connector 214 may be spaced apart from the first adapter assembly 210 by tubing fluidly coupling the first connector 214 with the first adapter assembly 210. Other configurations are also contemplated.

In some embodiments, the system 200 for priming a medical fluid system may comprise a second adapter assembly 220 comprising a second fluid connector 222 configured to interface with the sterile fluid source 20, a second connector 224 configured to fluidly couple with an outflow line of a medical fluid system, such as the outflow line 18 of the medical fluid system 10, and a gas release valve 226 configured to permit gas (e.g., air, oxygen, carbon dioxide, etc.) to pass therethrough and prevent fluid (e.g., liquid) from passing therethrough. Other configurations are also contemplated.

**In** some embodiments, the second fluid connector 222 may be coupled directly to the second adapter assembly 220. **In** some embodiments, the second fluid connector 222 may be monolithically formed with the second adapter assembly 220. **In** some alternative embodiments, the second fluid connector 222 may be spaced apart from the second adapter assembly 220 by tubing fluidly coupling the second fluid connector 222 with the second adapter assembly 220.

**In** some embodiments, the second connector 224 may be coupled directly to the second adapter assembly 220. **In** some embodiments, the second connector 224 may be monolithically formed with the second adapter assembly 220. **In** some alternative embodiments, the second connector 224 may be spaced apart from the second adapter assembly 220 by tubing fluidly coupling the second connector 224 with the second adapter assembly 220. Other configurations are also contemplated.

In some embodiments, the gas release valve 226 may be disposed upstream of the second fluid connector 222. In some embodiments, the gas release valve 226 may be disposed between the second fluid connector 222 and the second connector 224. In some embodiments, the gas release valve 226 may be coupled directly to the second adapter assembly 220 and/or the second connector 224. In some embodiments, the gas release valve 226 may be monolithically formed with the second adapter assembly 220 and/or the second connector 224. In some alternative embodiments, the gas release valve 226 may be spaced apart from the second adapter assembly 220 by tubing fluidly coupling the gas release valve 226 and/or the second connector 224 with the second adapter assembly 220. In some further alternative embodiments, the gas release valve 226 may be spaced apart from the second adapter assembly 220 by tubing fluidly coupling the gas release valve 226 with the second adapter assembly 220 and/or the second connector 224. Other configurations are also contemplated.

In some embodiments, the gas release valve 226 may be configured to vent gas 228 (e.g., air, oxygen, carbon dioxide, etc.) to atmosphere and direct fluid (e.g., liquid) to the second fluid connector 222. In some embodiments, the gas release valve 226 may be configured to vent gas 228 (e.g., air, oxygen, carbon dioxide, etc.) to atmosphere and direct fluid (e.g., liquid) into the sterile fluid source 20. In some alternative embodiments, the gas release valve 226 may be configured to vent gas (e.g., air, oxygen, carbon dioxide, etc.) into a collection container (not shown) and direct fluid (e.g., liquid) into the sterile fluid source 20. In some embodiments, the collection container may be separate from the sterile fluid source 20.

In some embodiments, fluid directed into the sterile fluid source 20 by the gas release valve 226 may be recirculated through a medical fluid system fluidly coupled thereto, such as the medical fluid system 10 fluidly coupled to the sterile fluid source 20 via the first fluid connector 212 and the second fluid connector 222. For example, as fluid from the sterile fluid source 20 passes into the inflow line 16, the fluid may displace and/or drive gas (e.g., air, etc.) disposed within the medical fluid system 10 and/or components thereof, such as the inflow line 16, the fluid pump 12, the oxygenator 14, the outflow line 18, etc., downstream toward and/or to the gas release valve 226. The gas release valve 226 may be configured to vent the gas (e.g., air, etc.) out of the medical fluid system 10 and/or the system 200 for priming a medical fluid system and direct the fluid into the sterile fluid source 20. The fluid may then be recirculated through the medical fluid system 10 so that additional fluid (and/or additional sterile fluid sources) is not required to prime, and/or properly remove gas (e.g., air, etc.) from, the medical fluid system 10, thereby reducing complexity for the user, as well as reducing cost and waste.

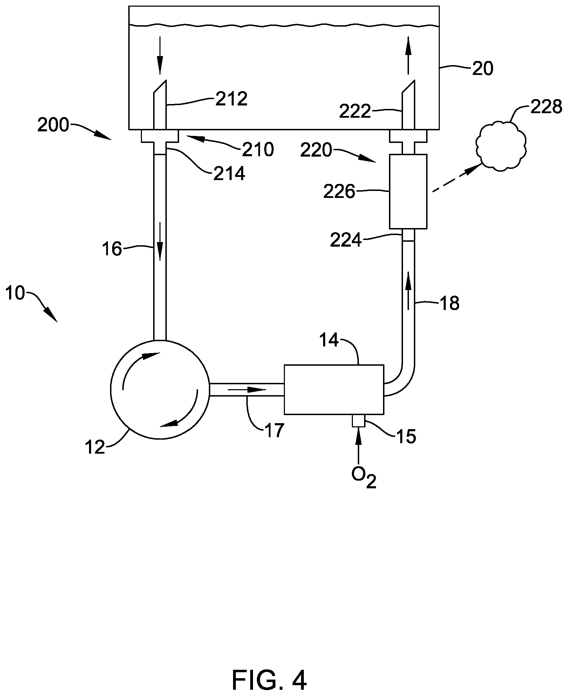

FIG. 4 illustrates selected aspects of the system 200 for priming a medical fluid system in use with the medical fluid system 10 and selected aspects of a method of priming a medical fluid system, such as the medical fluid system 10.

In some embodiments, the method of priming the medical fluid system 10 may comprise coupling the first connector 214 of the first adapter assembly 210 with the inflow line 16 of the medical fluid system 10. In some embodiments, the first connector 214 may comprise a female connector and coupling the first connector 214 of the first adapter assembly 210 with the inflow line 16 of the medical fluid system 10 may comprise inserting the inflow line 16 of the medical fluid system 10 into the first connector 214. In some embodiments, the first connector 214 may comprise a male connector and coupling the first connector 214 of the first adapter assembly 210 with the inflow line 16 of the medical fluid system 10 may comprise advancing and/or positioning the inflow line 16 of the medical fluid system 10 onto the first connector 214. In some embodiments, the inflow line 16 of the medical fluid system 10 may comprise a first mating connector configured to matingly couple with the first connector 214. Other configurations are also contemplated.

In some embodiments, the method of priming the medical fluid system 10 may comprise coupling the second connector 224 of the second adapter assembly 220 with the outflow line 18 of the medical fluid system 10. In some embodiments, the second connector 224 may comprise a female connector and coupling the second connector 224 of the second adapter assembly 220 with the outflow line 18 of the medical fluid system 10 may comprise inserting the outflow line 18 of the medical fluid system 10 into the second connector 224. In some embodiments, the second connector 224 may comprise a male connector and coupling the second connector 224 of the second adapter assembly 220 with the outflow line 18 of the medical fluid system 10 may comprise advancing and/or positioning the outflow line 18 of the medical fluid system 10 onto the second connector 224. In some embodiments, the outflow line 18 of the medical fluid system 10 may comprise a second mating connector configured to matingly couple with the second connector 224. Other configurations are also contemplated.

In some embodiments, the method of priming the medical fluid system 10 may comprise coupling the first fluid connector 212 of the first adapter assembly 210 for priming a medical fluid system with the sterile fluid source 20. In some embodiments, coupling the first fluid connector 212 with the sterile fluid source 20 may comprise inserting the first fluid connector 212 into the sterile fluid source 20. In some embodiments, coupling the first fluid connector 212 with the sterile fluid source 20 may comprise coupling the first fluid connector 212 with a first mating fluid connector of and/or in fluid communication with the sterile fluid source 20. Other configurations are also contemplated.

In some embodiments, the method of priming the medical fluid system 10 may comprise coupling the second fluid connector 222 of the second adapter assembly 220 for priming a medical fluid system with the sterile fluid source 20. In some embodiments, coupling the second fluid connector 222 with the sterile fluid source 20 may comprise inserting the second fluid connector 222 into the sterile fluid source 20. In some embodiments, coupling the second fluid connector 222 with the sterile fluid source 20 may comprise coupling the second fluid connector 222 with a second mating fluid connector of and/or in fluid communication with the sterile fluid source 20. Other configurations are also contemplated.

In some embodiments, the method of priming the medical fluid system 10 may comprise elevating the sterile fluid source 20 above the medical fluid system 10, thereby causing fluid from the sterile fluid source 20 to flow through the first adapter assembly 210 into the inflow line 16 of the medical fluid system 10, and through the medical fluid system 10 thereby pushing gas disposed therein downstream through the outflow line 18 to the gas release valve 226. In some embodiments, the sterile fluid source 20 may be disposed and/or elevated above the medical fluid system 10 before coupling the first fluid connector 212 with the sterile fluid source 20 such that gravity immediately causes fluid to flow from the sterile fluid source 20 toward and/or through the first adapter assembly 210 as soon as the first fluid connector 212 is coupled with the sterile fluid source 20 without any further interaction by the user/practitioner. For example, the sterile fluid source 20 may be a saline IV bag disposed and/or hanging on a support hook adjacent the patient when the first fluid connector 212 is coupled with the sterile fluid source 20. In some embodiments, the sterile fluid source 20 may be disposed and/or elevated above the medical fluid system 10 after coupling the first fluid connector 212 with the sterile fluid source 20 such that the user/practitioner must elevate the sterile fluid source 20 after coupling the first fluid connector 212 with the sterile fluid source 20 in order to initiate fluid flow from the sterile fluid source 20 toward and/or through the first adapter assembly 210. Other configurations are also contemplated.

In some embodiments, the gas release valve 226 may be configured to vent gas 228 (e.g., air, oxygen, carbon dioxide, etc.) to atmosphere and direct fluid (e.g., liquid) into the sterile fluid source 20. In some embodiments, the method of priming the medical fluid system 10 may comprise venting gas 228 (e.g., air, oxygen, carbon dioxide, etc.) to atmosphere or a collection container with the gas release valve 226. In some embodiments, the method of priming the medical fluid system 10 may comprise directing fluid (e.g., liquid) into the sterile fluid source 20 with the gas release valve 226. Other configurations are also contemplated.

In some embodiments, the method of priming the medical fluid system 10 may comprise recirculating the fluid directed into the sterile fluid source 20 by the gas release valve 226 through the medical fluid system 10. As discussed herein, as fluid flows from the sterile fluid source 20 through the medical fluid system 10, fluid is directed into the sterile fluid source 20 by the gas release valve 226, where the fluid may subsequently flow into the inflow line 16 of the medical fluid system 10, thereby recirculating the fluid in the sterile fluid source 20 through the medical fluid system 10.

The materials that can be used for the various components of the system for priming the medical fluid system and the various elements thereof disclosed herein may include those commonly associated with medical devices and/or systems. For simplicity purposes, the following discussion refers to the system. However, this is not intended to limit the systems, devices, components, and methods described herein, as the discussion may be applied to other elements, members, components, or devices disclosed herein, such as, but not limited to, the fluid connector(s), the connector(s), the reservoir, the gas release valve, the tubing, etc. and/or elements or components thereof.

In some embodiments, the device and/or components thereof may be made from a metal, metal alloy, polymer (some examples of which are disclosed below), a metal-polymer composite, ceramics, combinations thereof, and the like, or other suitable material.

Some examples of suitable polymers may include polytetrafluoroethylene (PTFE), ethylene tetrafluoroethylene (ETFE), fluorinated ethylene propylene (FEP), polyoxymethylene (POM), polyether block ester, polyurethane, polypropylene (PP), polyvinylchloride (PVC), polyether-ester, ether or ester based copolymers (for example, butylene/poly(alkylene ether) phthalate and/or other polyester elastomers), polyamide, elastomeric polyamides, block polyamide/ethers, polyether block amide (PEBA, for example available under the trade name PEBAX^{®}), ethylene vinyl acetate copolymers (EVA), silicones, polyethylene (PE), high-density polyethylene, low-density polyethylene, linear low density polyethylene, polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide, polysulfone, nylon, nylon-12, perfluoro(propyl vinyl ether) (PFA), ethylene vinyl alcohol, polyolefin, polystyrene, epoxy, polyvinylidene chloride (PVdC), poly(styrene-b-isobutylene-b-styrene), polycarbonates, polyisobutylene (PIB), polyisobutylene polyurethane (PIBU), polyurethane silicone copolymers, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear elastic and/or super-elastic nitinol; other nickel alloys such as nickel-chromium-molybdenum alloys, nickel-copper alloys, nickel-cobalt-chromium-molybdenum alloys, nickel-molybdenum alloys, other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys; platinum enriched stainless steel; titanium; combinations thereof; or any other suitable material.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A system for priming a medical fluid system, comprising:
a fluid connector configured to interface with a sterile fluid source; and
a fluid reservoir downstream of and in fluid communication with the fluid connector, the fluid reservoir comprising:
a first connector configured to fluidly couple with an inflow line of the medical fluid system;
a second connector configured to fluidly couple with an outflow line of the medical fluid system; and
a gas release valve configured to permit gas to pass therethrough and prevent fluid from passing therethrough.

2. The system of claim 1, wherein the fluid reservoir further comprises a one-way baffle disposed between the second connector and the first connector.

3. The system of claim 2, wherein the one-way baffle permits fluid to flow therethrough from a second connector side of the one-way baffle toward a first connector side of the one-way baffle.

4. The system of claim 3, wherein the gas release valve is upstream of the one-way baffle.

5. The system of claim 3, wherein the one-way baffle permits fluid to flow therethrough only from the second connector side of the one-way baffle toward the first connector side of the one-way baffle.

6. The system of any one of the preceding claims, wherein the fluid reservoir is configured to recirculate fluid from the sterile fluid source through the medical fluid system fluidly coupled thereto.

7. The system of any one of the preceding claims, wherein the gas release valve is configured to vent gas to atmosphere and direct fluid into the fluid reservoir.

8. The system of any one of the preceding claims, further comprising a fluid supply line extending from the first fluid connector to the fluid reservoir.

9. The system of any one of the preceding claims, wherein the medical fluid system comprises a fluid pump and an oxygenator.

10. The system of any one of the preceding claims, wherein the sterile fluid source is a saline bag.

11. A method of priming a medical fluid system, comprising:
coupling a first connector of a fluid reservoir with an inflow line of the medical fluid system;
coupling a second connector of the fluid reservoir with an outflow line of the medical fluid system;
coupling a first fluid connector with a sterile fluid source, wherein the first fluid connector is in fluid communication with the fluid reservoir; and
elevating the sterile fluid source above the medical fluid system, thereby causing fluid from the sterile fluid source to flow through the fluid reservoir into the inflow line of the medical fluid system, and through the medical fluid system thereby pushing gas disposed therein downstream through the outflow line to a gas release valve;
wherein the gas release valve is configured to vent gas to atmosphere and direct fluid into the fluid reservoir.

12. The method of claim 11, further comprising recirculating the fluid in the fluid reservoir through the medical fluid system.

13. A system for priming a medical fluid system, comprising:
a first adapter assembly comprising a first fluid connector configured to interface with a sterile fluid source and a first connector configured to fluidly couple with an inflow line of the medical fluid system; and
a second adapter assembly comprising a second fluid connector configured to interface with the sterile fluid source, a second connector configured to fluidly couple with an outflow line of the medical fluid system, and a gas release valve configured to permit gas to pass therethrough and prevent fluid from passing therethrough.

14. The system of claim 13, wherein the gas release valve is disposed upstream of the second fluid connector, and the gas release valve is disposed between the second fluid connector and the second connector.

15. The system of claim 13 or 14, wherein the gas release valve is configured to vent gas to atmosphere and direct fluid to the second fluid connector.
